(19)

Europäisches
Patentamt
European
Patent Office
Office européen
des brevets

(11) **EP 4 230 126 A1**

(12) **EUROPEAN PATENT APPLICATION**
published in accordance with Art. 153(4) EPC

(43) Date of publication:
**23.08.2023 Bulletin 2023/34**

(21) Application number: **21881396.2**

(22) Date of filing: **19.10.2021**

(51) International Patent Classification (IPC):
***A61B 5/03*** (2006.01)     ***A61B 5/00*** (2006.01)

(86) International application number:
**PCT/BR2021/050456**

(87) International publication number:
**WO 2022/082289 (28.04.2022 Gazette 2022/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.10.2020 BR 102020021338**

(71) Applicant: **Braincare Desenvolvimento E
Inovação Tecnológica S.A.
13562-400 São Carlos, São Paulo (BR)**

(72) Inventors:
• **ANDRADE, Rodrigo De Albuquerque Pacheco
13561-200 São Carlos (BR)**

• **JUNIOR, Deusdedit Lineu Spavieri
13562-240 São Carlos (BR)**
• **OLIVEIRA, Sérgio Mascarenhas
14093-210 Ribeirão Preto (BR)**
• **MIYAZAKI, Caio Kioshi
13564-210 São Carlos (BR)**
• **OSHIRO, Helder Eiki
13561-090 São Carlos (BR)**

(74) Representative: **Grünecker Patent- und
Rechtsanwälte
PartG mbB
Leopoldstraße 4
80802 München (DE)**

(54) **SYSTEM AND METHOD FOR MULTI-CHANNEL DETECTION AND MONITORING OF
INTRACRANIAL PRESSURE, AND MULTI-CHANNEL DEVICE**

(57)     The present invention describes a non-invasive multichannel intracranial pressure detection and monitoring system and method. The invention presents a solution for multichannel detection of volumetric variations of the skull and processing the signals coming from the plurality of sensors in order to obtain the result of the intracranial pressure of a user with greater precision. For this, the present invention proposes a plurality of sensors, positioned on the user's head, which communicate with a processing unit, the sensors being arranged in a fixation structure. These sensors also have auxiliary sensors, which make it possible to contribute to the detection result. The present invention is in the fields of medicine, biomedicine, neuroscience, measurement of physical quantities and electrical engineering.

FIG. 1

EP 4 230 126 A1

## Description

### Field of the Invention

The present invention describes a solution for the non-invasive multichannel intracranial pressure detection and monitoring, seeking greater precision in the detected results and allowing greater flexibility in the detection of signals, since it allows both the global analysis and the localized analysis, in addition to enabling the detection of additional signals by means of a biometric unit. The present invention is in the fields of medicine, biomedicine, neuroscience, measurement of physical quantities and electrical engineering.

### Background of the invention

Conventional intracranial pressure (ICP) monitoring methods existing in the prior art include penetration into the skull and insertion through it and dura mater of a catheter to perform the measurement. This highly invasive procedure includes risks of precipitating an intracranial hematoma, aggravating cerebral edema, damaging the parenchyma, intracerebral hemorrhage and promoting intracranial infection, the latter being the most common complication in patients monitored for more than a week after surgery. Considering all the disadvantages mentioned, the need to monitor ICP with at least a minimally invasive method that eliminates the complications generated by penetration within the skull is of extreme clinical importance, as it is opening up new fields of research on this important physiological parameter. Therefore, there is a need to develop devices capable of detecting the volumetric variation of the patient's skull, allowing the correct result of the ICP morphology to be obtained.

There are also some technologies that make it possible to measure ICP in a non-invasive way, however most of these technologies perform the measurement through concepts of frequency response/impulse response, where, for those skilled in the art, it deals with the injection of a known signal in the cranial box and the reading of this signal after traveling through the cranial box and, with that, the ratio of the injected signal to the received signal is made. This is the case of patent WO9202174 and US2009234245, in addition to many others. From this, standardization and calibration are carried out so that, through eventual modifications in the read signal, ICP measurement can be carried out. These concepts, however, perform the ICP measurement in a punctual way, that is, in only one region of the patient's skull, so that if there is an increase in pressure in another region, the system is not able to identify it.

Additionally, there are systems that measure the blood pressure level or cerebrospinal fluid circulating in the patient's head, where many of these technologies use accelerometers to perform this measurement. Thus, the system relates the measured pressure to the patient's intracranial pressure. However, these systems are liable to loss in signal measurement, since they do not directly measure intracranial pressure. This type of system is verified in patent US2016081608.

There are, therefore, systems that measure ICP through volumetric variation/skull deformation, as is the case of patents WO2013041973 and WO2019087148. In these solutions, the systems use sensors that identify the volumetric variation of the skull and, therefore, attribute this variation to the morphology of the ICP. Such systems are based on the fact that the skull is not an extremely rigid and fixed structure, unlike the others mentioned above, so that the cranial box performs deformations based on the patient's intracranial pressure. These solutions, however, do not bring the possibility of multichannel acquisition morphology signal of the ICP, as this signal is extracted concomitantly from different regions of the patient's skull.

Thus, from what can be seen from the researched literature, no documents were found anticipating or suggesting the teachings of the present invention, so that the solution proposed herein has novelty and inventive activity against prior art.

### Summary of the Invention

In this way, the present invention aims to solve the prior art problems from the integration of multiple signals detected by a plurality of biometric units (1) arranged in the head of a user in order to produce several possibilities of visualization of the result from the detected ICP morphology. The biometric units (1) are arranged in distinct regions on the patient's head and provide the measurements concomitantly to a processing unit (2).

In this sense, the present invention aims to improve the accuracy of data acquisition for the capture of intracranial pressure signals in a non-invasive way, in addition to enabling the collection and processing of other parameters related to the user, from the biometric units positioned in a global way, allowing to carry out different analyzes based on these parameters. Visualization of the morphology of the ICP detected by several sensors in different locations around the skull enables greater precision and reliability in the results obtained.

Additionally, the present invention provides the biometric units (1) composed with at least one sensor for acquiring an additional signal to the volumetric variation/cranial deformation so that this additional signal is compensated in the calculation of the ICP or is used in the quality verification of the channel, making it possible to check any interference in the measurement.

In a first object, the present invention shows a system for a non-invasive multichannel intracranial pressure detection and monitoring of a user from cranial deformation, wherein the system comprises: a device for detection and monitoring of intracranial pressure that comprises a plurality of biometric units (1) positionable on the user's head, which communicates with at least one processing unit (2), said processing unit (2) comprising a processor (2.1) communicating with a communication interface (2.2), for reading and processing signals from each biometric unit (1); and a receiver operating in communication with the processing unit (2) of the intracranial pressure detection and monitoring device.

In a second object, the present invention shows a non-invasive multichannel intracranial pressure detecting and monitoring device comprising: a plurality of biometric units (1) communicating with at least one processing unit (2), the said processing unit (2) comprising a processor (2.1) communicating with a communication interface (2.2) for reading and processing signals from each biometric unit (1); and at least one fixation structure (3) positionable on a user's head, wherein the plurality of biometric units (1) are radially distributed along the fixation structure (3).

In a third object, the present invention shows a method for non-invasive multichannel intracranial pressure detection and monitoring of a user from a cranial deformation, which comprises the steps: i) detecting of multiple signals related to the user's cranial deformation by means of an intracranial pressure detection and monitoring device comprising a plurality of biometric units (1) positionable on the user's head; ii) receiving of the multiple signals related to the cranial deformation by a communication interface (2.2), one or more signals related to the cranial deformation being sent to a processor (2.1), wherein the communication interface (2.2) and the processor (2.1) are comprised in a processing unit (2); and iii) processing, by the processor (2.1), one or more signals related to the cranial deformation generating intracranial pressure signals.

These and other objects of the invention will be immediately appreciated by those skilled in the art and will be described in detail below.

**Brief Description of the Figures**

In order to better define and clarify the content of the present patent application, the following figures are showed:

Fig. 1 shows an embodiment of the non-invasive intracranial pressure detecting device.

Fig. 2 shows an embodiment of the intracranial pressure detection device adjusted by the fixation structure (3).

Fig. 3 shows an embodiment of the intracranial pressure detection device configured by biometric units (1) containing a plurality of sensors.

Fig. 4 shows a side view of a possible embodiment of the biometric unit.

Fig. 5 shows a profile view of a possible embodiment of the biometric unit.

Fig. 6 shows a possible embodiment of the biometric unit highlighting the positions of the auxiliary sensors.

Figs. 7a and 7b show the operation of the sensor that performs the plethysmography.

Fig. 8 shows the different curves of the signals obtained from plethysmography sensors (1.4) in different positions of the user's head.

Fig. 9.a shows a possible embodiment with a cap for allocating a plurality of biometric units (1). Fig. 9.b shows another embodiment with a cap, where the biometric units (1) are interconnected. Fig. 9.c shows an embodiment of the cap with an upper structure that presses the biometric units (1).

Figs. 10.a to 10.h show embodiments of biometric units containing a plurality of sensors having different displacement transducer alternatives.

Fig. 11 shows a possible embodiment of the system topology highlighting the energy and information flowchart between the blocks.

Fig. 12 shows a possible embodiment of the system architecture emphasizing a plurality of channels and analog/digital converters.

Fig. 13 shows a possible embodiment of the system architecture emphasizing the plurality of channels and analog/digital converters in a configuration that allows the expansion of the number of channels.

Fig. 14 shows a block diagram highlighting the use of a multichannel processing performed through the information acquisition from ambient sensors and biometric units.

Fig. 15 shows several ICP morphology curves obtained in different positions of the human head.

Fig. 16 shows an ICP morphology curve obtained by a non-invasive measurement resulting from multichannel processing.

Fig. 17 shows a representative flowchart of an embodiment for calculating the channel quality index.

Fig. 18 shows an exemplary graph representing the influence of the plethysmography signal on the displacement signal.

Fig. 19.a shows an exemplary block diagram for the acquisition of two signals and Fig. 19.b shows the acquired waveforms.

Fig. 20.a shows an exemplary block diagram for compensating the delay between two signals and Fig. 20.b shows the waveforms of the resulting signals.

Fig. 21.a shows an exemplary block diagram for resampling and interpolation of one of the signals and Fig. 21.b shows the waveforms of the resulting signals.

## Detailed Description of the Invention

The following descriptions are presented by way of example and not limiting the scope of the invention and will make the object of the present patent application more clearly understood.

For the purposes of the present invention, "volumetric variation of the skull" or "cranial deformation" refer to the deformation suffered by the cranial box from the intracranial pressure variation of a user. It should be noted that this deformation can be both positive and negative, that is, both expansion and retraction can occur.

In a first object, the present invention shows a system for a non-invasive multichannel intracranial pressure detection and monitoring of a user from the cranial deformation, wherein the system comprises: a device for detecting and monitoring the intracranial pressure that comprises a plurality of biometric units (1) positionable on the user's head, which communicates with at least one processing unit (2), said processing unit (2) comprising a processor (2.1) communicating with a communication interface (2.2) for reading and processing signals from each biometric unit (1); and a receiver operating in communication with the processing unit (2) of the intracranial pressure detection and monitoring device.

For the purposes of the present invention, "multichannel" refers to the fact that the solution proposed herein operates with more than one sensor device capable of capturing signals related to intracranial pressure and, therefore, obtaining the signal of cranial deformation in different regions of the user's head. These signal acquisitions can occur either all at the same time, operating in parallel, or operating synchronously, with one or more sensors operating sequentially.

The plurality of biometric units (1) is defined by containing multiple sensors, wherein at least one of which is capable of detecting the volumetric variation of the skull and transforming the mechanical force exerted on the sensory apparatus into an electrical signal, which act continuously, radially spread around the along the user's head, and can be equally spaced or positioned at specific points for the detection, in several regions of the skull, of punctual intracranial pressure. In one embodiment, the biometric units (1) comprise displacement sensors (1.1). These displacement sensors (1.1) can perceive mechanical load, both positive and negative, and transduce the mechanical energy resulting from the ICP variation into electrical energy through capacitive, inductive, optical, piezoelectric, resistive and/or through ultrasound, fiber optics, interferometrics, radar, cranial bioimpedance variation, or any combination of these technologies.

In one embodiment, the non-invasive multichannel intracranial pressure detecting device is provided with a plurality of biometric units (1) distributed along a fixation structure (3), which is capable of being adjusted around the skull in a way non-invasive.

In one embodiment, the biometric units (1) comprise a contact sensor (1.2). One function of this sensor is to provide information about the interface and quality of contact of the respective biometric unit (1) with the part of the head receiving the displacements, verifying, for example, the contact with human skin. This procedure reduces errors or inaccuracies caused by poorly established contact due to the presence of intermediate materials between the skin and the biometric unit (1), such as hair. In one embodiment, the contact sensor (1.2) is a temperature sensor, where, for example, from the temperature data obtained, it is possible to verify whether the temperature is close to the human skin expected temperature. By means of the temperature sensor, it is also possible to measure and calculate the temperature variations between the multiple channels of the system, in order to check the temperature at different points of the patient's head. In another embodiment, the contact sensor (1.2) is an electrical biopotential sensor, which operates with a faster and more dynamic response to obtain the relative electrical potential between the system channels (of natural origin of the bodies). In another embodiment, the contact sensor (1.2) is a continuous electrical bioimpedance sensor, where a low-intensity electric current is applied between the channels to calculate the relative impedance of the channels, varying in time, to perceive the variation of the relative contact between the displacement sensors (1.1) of the biometric units and the user's head.

In one embodiment, the plurality of biometric units (1) comprises a plethysmography sensor (1.4). This sensor is equipped with a light emitter (1.4a) and a photodetector (1.4b), with the function of detecting peripheral extra-cranial blood circulation (6) in specific positions of the user's head, and thus using its data, for example, to exclude possible noises in the data obtained by the displacement sensors (1.1), thus obtaining a better ICP morphology. The sensor operation depends on the light reflection at a specific wavelength in the oxy-hemoglobin cells that are present in the blood, and this reflection is acquired by the photodetector (1.4b) and is proportional to the number of these cells.

In one embodiment, the plurality of biometric units (1) comprises an inertial sensor (1.5). The sensor is a nine-degrees freedom IMU (1.5), featuring three accelerometers, which are capable of measuring linear velocity variations in the three orthogonal directions that define space through a process of transforming kinetic energy into electrical energy; three gyroscopes, which are capable of measuring rotations in the three orthogonal directions that define space through variations in angular movement; and three magnetometers, which are capable of measuring variations in the magnetic field in the three orthogonal directions defining the space through physical phenomena, with emphasis on the Hall effect, for example.

In one embodiment, the plurality of biometric units (1) comprises a plurality of additional embedded sensors, whose function is to monitor the ambient conditions having complementary data to optimize the ICP morphology measurements. This plurality of embedded sensors, for example purposes, contains at least one of a hygrometer, capable of measuring air humidity; a barometer, capable of measuring the atmospheric pressure of the patient's monitoring environment; a thermometer, capable of measuring the ambient temperature; an embedded inertial sensor with a topology similar to that used by the inertial sensor (1.5) showed in the biometric units (1); or a combination thereof.

Said processing unit (2) comprises a processor (2.1) and a communication interface (2.2). Said communication interface (2.2) is any hardware or software component being implemented in the processor itself (2.1) or as an individual component, capable of receiving signals from all biometric units (1) and forwarding to the processor (2.1). In one embodiment, the communication interface (2.2) receives all signals from the displacement sensors (1.1) of the biometric units (1). In this embodiment, the signals from the auxiliary sensors of the biometric units (1) are directly forwarded to the processor or through another communication interface.

In one embodiment, the processor (2.1), by means of a selector element, selects one or more signals from the biometric units (1). With this, the selector element allows the signals to be globally, locally, or punctually processed and/or analyzed. Globally it refers to the fact that all signals are read and processed; locally refers to just a few signals in one or more specific regions of the skull; and punctually refers to signals coming from a single sensor. Additionally, the selector element is controlled by the processor (2.1), so that the processor (2.1) sends a channel selection command to said selector element. Also, this channel selection command can be issued by a component external to the device or system, for example, by the receiver itself, or by a system operator.

In one embodiment, said selector element may be a hardware or software component capable of selecting one or more channels. In one embodiment, the selector element is a software component implemented through programming in the processor (2.1) itself, which selects the channels to be read or processed upon receipt of an instruction from the processor (2.1). For example, the selector element is a channel enabler/disabler. In another embodiment, said selector element is a pre-configured digital filter for channel separation.

In one embodiment, the processor (2.1) receives a plurality of signals from the biometric units (1) and from environmental sensors as digital signals. Therefore, in this embodiment, the communication interface (2.2) comprises a plurality of analog/digital converters, which can be arranged in different topologies in order to receive and convert the signals coming from all the biometric units (1). The way the topology is established defines the number of biometric units (1) that are simultaneously acquired by the processing unit (2), enabling greater information accuracy.

The processor (2.1) aims to receive all the signals generated by the plurality of biometric units (1) and by the embedded environment sensors, to process the signals received by the plurality of displacement (1.1), contact (1.2), plethysmography (1.4), inertial (1.5) and environment sensors on several ways in order to result in the measurement of the volumetric variation of the skull according to the operator's need for a system for monitoring and managing intracranial pressure. In one embodiment, the signals received from the plurality of biometric units (1) are processed in parallel by the processor (2.1). In another embodiment, the signals received from the plurality of biometric units (1) are serially processed by the processor (2.1). In another embodiment, only the signals defined by the operator of the intracranial pressure monitoring and detection system are processed by the processor (2.1). The processing of signals selected by the system operator includes individual and joint mathematical and statistical manipulations in order to obtain an optimized ICP morphology curve. Additionally, the processing performed refers to the generation of one or more waveforms related to the signals provided by the biometric units (1).

In one embodiment, the processing unit (2) performs the acquisition of one or more signals from one or more sensors present in a plurality of biometric units (1) and performs the individual processing of each of the channels, wherein each channel is considered to be at least one biometric unit (1). These processed signals result in an optimization of the data of interest for monitoring a patient's ICP morphology, and are sent through a transmitter operating in a predetermined communication protocol.

In one embodiment, the processor (2.1) comprises a signal compensation tool, which attenuates, from the intracranial pressure signal, signals from the contact sensor (1.2), the plethysmography sensor (1.4) and/or the inertial sensor (1.5). In this sense, the processor (2.1) uses contact, plethysmography and movement signals to attenuate any noise that may interfere with the cranial deformation signal, making a compensation.

In one embodiment, the processor (2.1) or the receiver comprises a channel signal quality index generation tool, wherein said tool generates a quality index from signals coming from the displacement sensor (1.1), the contact sensor (1.2), plethysmography sensor (1.4) and/or inertial sensor (1.5) for each biometric unit (1) of the intracranial pressure detection and monitoring device. Thus, an index is assigned to each channel that represents the contribution that the contact, plethysmography and/or movement signals make to the general volumetric variation of the patient's skull, in addition to verifying whether the detected displacement signal has any interference component caused, for example, due to a bad positioning of the biometric unit (1). The index of each channel is then multiplied by the displacement signals, detected by the displacement sensor (1.1), of the respective channel, so that it is possible to verify the quality of the measurement of the respective channel.

In one embodiment, said processing unit (2) has a transmitter, which communicates with the processor (2.1) in order to receive the processed cranial deformation signals and send them to the receiver. In one embodiment, this transmitter is a wired communication board. In another embodiment, the transmitter is a wireless communication board, which transmits information through predetermined protocols.

In one embodiment, the receiver is a component adapted to receive data from the processing unit (2), which may be a monitoring unit with a display for display the data. Additionally, the receiver can be a cloud database unit, which receives signals from the processing unit (2). Also, the signals sent by the processing unit (2) can be provided to multiple receivers, for example, local receivers in a hospital/laboratory environment, and cloud receivers, such as the database.

It is also an object of the present invention a non-invasive multichannel intracranial pressure detecting and monitoring device, comprising: a plurality of biometric units (1) communicating with at least one processing unit (2), being said processing unit (2) comprising a processor (2.1) communicating with a communication interface (2.2) for reading and processing signals from each biometric unit (1); and at least one fixation structure (3) positionable on a user's head, wherein the plurality of biometric units (1) are radially distributed along the fixation structure (3). The distribution of biometric units (1) in the fixation structure (3) can occur perimetrically or superficially.

In one embodiment, said processing unit (2) is positioned on the fixation structure (3). In one embodiment, the plurality of biometric units (1) communicate with the processing unit (2) by means of electrical wires that are housed in the fixation structure (3) itself. In one embodiment, the communication between the plurality of biometric units (1) and the processing unit (2) occurs wirelessly.

In one embodiment, the plurality of biometric units (1) comprises a support (1.3). This support has the function of making the connection between the biometric unit (1) and the fixation structure (3) in such a way that there is no, or minimizes, interference in the acquisition of sensors existing in the biometric unit (1) and that a constant stability in relation to the fixation structure (3) is established.

In one embodiment, the fixation structure (3) comprises a tape interconnected through an adjustment device (4) and at least one tightening sensor. The fixation structure (3) aims to be adjustable to the shape of the skull, additionally having greater sensitivity, while preserving the integrity of the skull in situations of patient fragility. The adjustment device (4) together with a tightening sensor is capable of serving as a metric to ensure that the initial pressure set in the head meets the minimum initial conditions for correct monitoring of the ICP. In another embodiment, the fixation structure (3) is composed of an elastic or adjustable material in the shape of a cap, which allows the positioning of a plurality of biometric units (1) in specific positions on the head. In both embodiments, the fixation of the biometric units (1) to the fixation structure (3) is carried out through a support (1.3) associated with the biometric unit (1), to provide greater stability during use, to avoid changes in operating rest conditions and tamper the results of a monitoring.

In one embodiment, the biometric unit (1) comprises the displacement sensor (1.1) attached to at least one unit support (1.3) which is associable to the fixation structure (3). This configuration makes it possible for the greater stability provided by the unit support (1.3) to be also transferred to the displacement sensor (1.1).

Additionally, the biometric unit comprises at least one contact sensor (1.2), at least one plethysmography sensor (1.4) and at least one inertial sensor (1.5). Said sensors being previously defined.

In one embodiment, the contact sensor (1.2) and the plethysmography sensor (1.4) are arranged in a first region (A), this being the region closest to the user's head. For example, the first region (A) is in contact with the user's head. This arrangement is carried out to promote that both the contact sensor (1.2) and the plethysmography sensor (1.4) are in contact or close to the skin. The contact sensor (1.2) allows check the interface and quality of the contact of the biometric unit (1) with the part of the head that receives the displacements, being, for example, a temperature sensor, in order to verify if the temperature is close to the expected human skin temperature, where this procedure reduces errors or inaccuracies caused by poorly established contact due to the presence of intermediate materials between the skin and the biometric unit (1), such as hair. The plethysmography sensor (1.4) depends on the light reflection at a specific wavelength in the oxyhemoglobin cells that are present in the blood and, aiming at greater precision in the results, it is arranged close to the patient's head.

In one embodiment, the unit support (1.3) is arranged opposite the first region (A), so that the displacement sensor (1.1) is arranged between the first region (A) and the unit support (1.3).

In one embodiment, the displacement sensor (1.1) is positioned between two flat cylindrical ends, being one the mobile base and the unit support (1.3). Both the contact sensor (1.2) and the plethysmography sensor (1.4) are arranged in said mobile base, which is closer to the first region (A). In a realized configuration, the emitter, and the photodetector of the plethysmography sensor (1.4) are arranged in the center of the most external surface of the mobile base, while the contact sensor (1.2) is arranged in a ring shape on the external part of the mobile base. Also, the inertial sensor (1.5) is placed on the unit support (1.3), being closer to the fixation structure (3).

It is also an object of the present invention, a non-invasive multichannel intracranial pressure detection and monitoring method of a user, from a cranial deformation, which comprises the steps: i) detecting of multiple signals related to the cranial deformation of the user, by means of an intracranial pressure detection and monitoring device comprising a plurality of biometric units (1) positionable on the user's head; ii) receiving the multiple signals related to the cranial deformation by a communication interface (2.2), one or more signals related to the cranial deformation being sent to a processor (2.1), wherein the communication interface (2.2) and the processor (2.1) are comprised in a processing unit (2); and iii) processing, by the processor (2.1), one or more signals related to the cranial deformation generating intracranial pressure signals.

In one embodiment, the step of detecting multiple signals is performed by means of the previously described multichannel detection device. The detection of multiple signals can be performed in parallel, that is, with all the biometric units (1) performing the detections concomitantly, or serially, in a synchronized way.

In one embodiment, the communication interface (2.2) receives all signals from the displacement sensors (1.1) of the biometric units (1). In this embodiment, the signals from the auxiliary sensors of the biometric units (1) are forwarded directly to the processor or through another communication interface.

Additionally, the method comprises a channel selection step, by means of a selector element, wherein the processor (2.1) selects one or more signals related to the cranial deformation coming from one or more biometric units (1). This channel selection step allows the signals to be globally, locally, or punctually processed and/or analyzed. Also, the channel selection step can be performed at the request of a system operator or autonomously, by the processor (2.1) itself through prior configuration, wherein the processor (2.1) can be configured to select one or more channels from a condition detected by one of the sensors of the biometric unit (1).

Additionally, the method of the present invention comprises a step of generating a channel signal quality index being generated for each of the multiple signals relating to the cranial deformation, wherein said channel signal quality index is generated from displacement signals, contact signals, plethysmography signals and/or movement signals coming from the plurality of biometric units (1). In one embodiment, this index is generated by the processor (2.1), by the receiver or by an external processing unit linked to the receiver.

In one embodiment, the quality index is assigned to each channel representing the contribution that the contact, plethysmography and/or movement signals make to the general volumetric variation of the patient's skull, in addition to verifying whether the detected displacement signal has any component of interference. The index of each channel is then multiplied by the displacement signals detected by the displacement sensor (1.1), of the respective channel, so that it is possible to verify the quality of the measurement of the respective channel. In view of this index, the processor (2.1) or a system operator can select the channels, through the selector element, that have a higher detection quality, that is, that have a lower contribution from contact, plethysmography and/or movement signals. From this, it is possible to obtain the measurement of the cranial deformation in the points that have less interference from these signals.

In one embodiment, the generation of intracranial pressure signals in the processing step refers to the generation of one or more waveforms related to the signals provided by the biometric units (1). The generation of waveforms and signals of the ICP morphology, from the displacement signals, can follow the concepts already used in the prior art.

Moreover, in this processing step it is possible to apply mathematical/statistical tools for the evaluation of multiple signals. For example, it is possible to average the signals detected by all biometric units (1) or just a predefined part. Furthermore, it is possible to extract parameters referring to different regions of the skull, based on the signals acquired by the biometric units of the respective regions.

The present invention, unlike the systems known from the prior art, through the non-invasive way multichannel intracranial pressure device, it allows an adjustment for a correct monitoring of the intracranial pressure, a detection of the cranial expansion in several points of the skull, a way of to obtain the intracranial pressure with high precision, a processing of the detected signals that results in an accurate pressure and a fluid and continuous transmission of data. In addition, the present solution also aims to allow the location of the region that generates the greatest volumetric variation of the skull.

**Example**

The examples shown herein are intended only to exemplify one of the many ways to carry out the invention, but without limiting its scope.

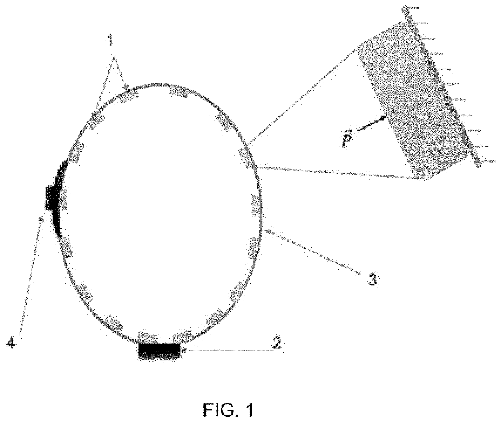

Fig. 1 shows one of the numerous variant embodiments derived from the present invention. The plurality of biometric units (1) of the intracranial pressure detection device is formed by displacement sensors (1.1), which are arranged in such a way as to be equally spaced and perimeter along the fixation structure (3). The patient's skull applies a load to each sensor so that the force (P) exerted against the displacement sensor (1.1) has direction and sense to form a normal vector to the sensor. It should be noted that said force (P) can be both positive and negative, that is, the skull can expand its volume or retract its volume.

Normal vector is defined as a force (P) perpendicular to the impact area so that all pressure exerted is detected and converted into an electrical signal.

The processing unit (2) has a communication interface (2.2), in this case a multisensor analog input conditioner (Multisensor analog front end), which receives in parallel all the signals generated by the plurality of biometric units (1), processor (2.1) that processes all the signals generated by the deformation of the skull, and a transceiver to receive and send the generated data through a wireless communication with the receiving system.

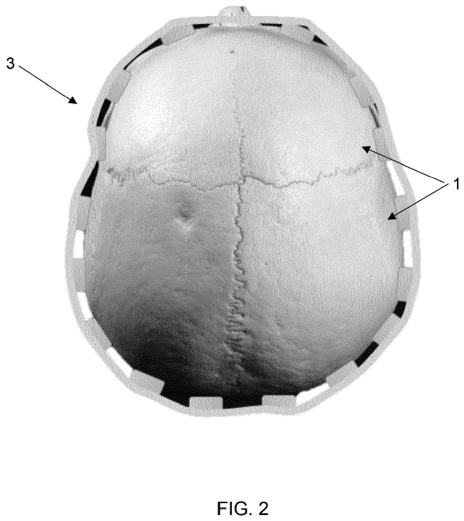

The fixation structure (3) consists of a material that transmits all the mechanical energy generated by the deformation of the skull to the plurality of biometric units (1). The adjustment device (4) and the tightness sensor are used as an initial condition reference and as a metric system by adjusting the fixation structure (3) around the cranial perimeter as shown in Fig. 2.

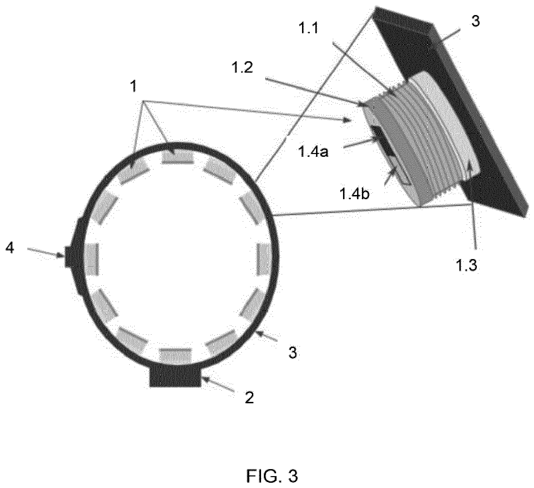

In one embodiment, Fig. 3 shows biometric units (1) composed of a variety of sensors, including plethysmograph and contact sensors, permeating the fixation structure (3). The use of biometric units (1) containing a larger number of sensors provides a better obtainment of information, enabling the realization of an estimate closer to the reality of the ICP morphology. In the example, the fixation structure (3) takes the form of a malleable and adjustable strap, making it possible to adapt and position the device on a user's head, and for a more efficient transmission of mechanical energy resulting from ICP variations, while maintaining the integrity of the patient in situations of cranial fragility.

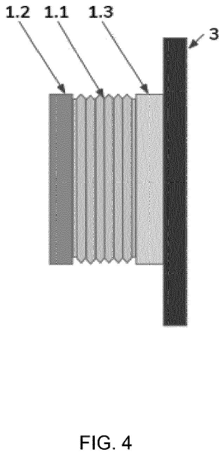

Fig. 4 shows a side view of the biometric unit, consisting of a displacement sensor (1.1), a mobile base, where the contact sensor (1.2) is positioned, and a unit support (1.3) for attachment to the fixation structure (3), so that the displacement sensor (1.1) is placed between the mobile base and the unit support (1.3). In this example, the contact sensor (1.2) is a temperature sensor, having the form of a ring, and captures the temperature of the region where it is touching. The displacement sensor (1.1) has a specialized format to transform the variations of mechanical energy that occur in the skull into electrical energy, through different types of transducers that can be resistive, inductive, capacitive, piezoelectric, and obtained through interferometric methods, light signal variation through optical fiber, radar-type systems and ultrasonic wave reception.

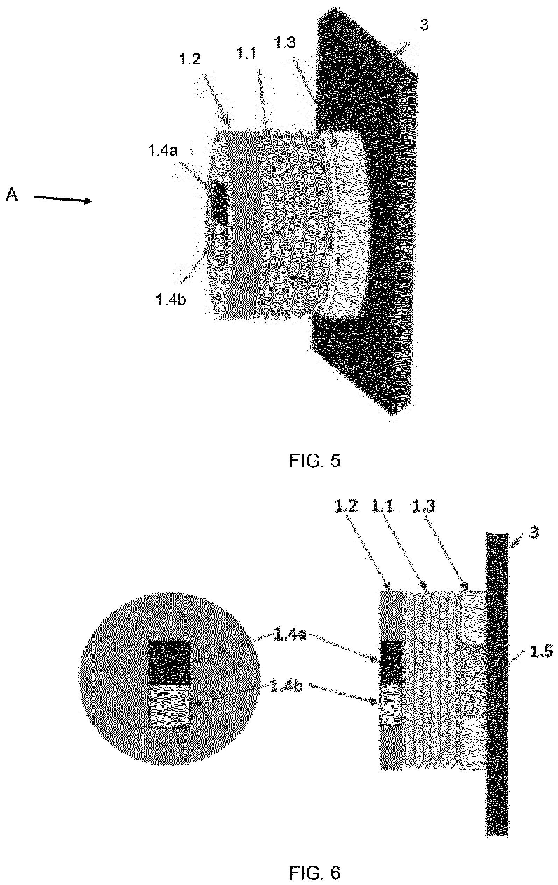

Fig. 5 is a profile view of the described biometric unit, highlighting the position of the externally visible sensors, indicating the presence of region (A), being the region closest to the user's head. Such an arrangement allows both the temperature sensor (1.2) and the plethysmography sensor (1.4) to be arranged in the region closest to the head, preferably - but not necessarily - in contact with the user's skin.

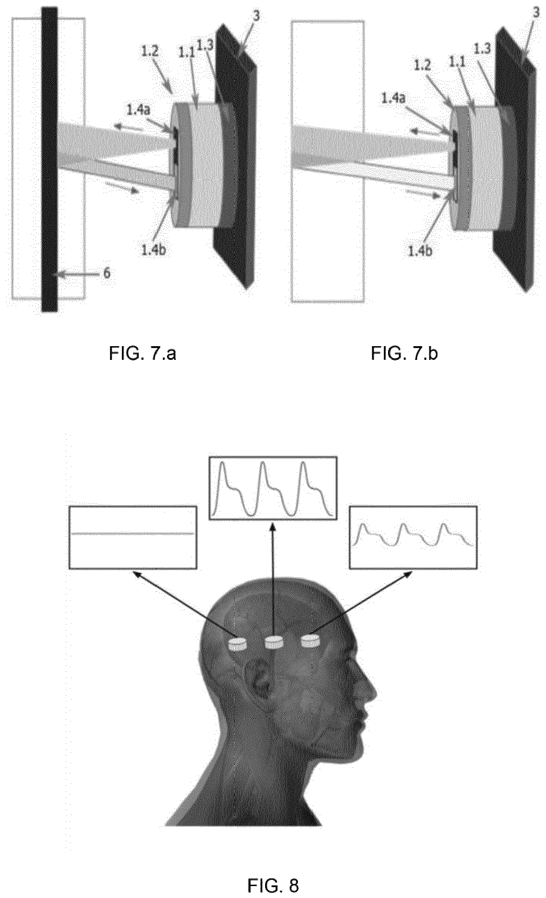

Fig. 6 shows the choice of locations for plethysmograph sensors, which perform the emission and acquisition of light at a defined wavelength to capture the presence of peripheral extra-cranial blood circulation (6), a procedure illustrated by figures 7.a and 7.b. In these figures, the amount of oxy-hemoglobin present in peripheral extra-cranial blood circulation (6) can be estimated through the reflection of an amount of light previously emitted by the light emitter (1.4a) and acquired by the photodetector (1.4b), which is proportionately larger as the amount of cells of the mentioned type increases. In the example, the light emitter is an LED.

Still in Fig. 6 it is possible to observe the presence of an inertial sensor (1.5), being a 9-degrees of freedom IMU in the unit support (1.3) of the biometric unit. In the embodiment, this sensor is composed of 3 accelerometer channels, 3 gyroscope channels and 3 magnetometer channels, which carry out measurements of the position and movement of the head and of each of the biometric units (1) wherein they are located. This component is essential to correlate limb and sensor movements with the obtained ICP morphology signals, thus validating and correcting the data and performing more accurate analyses.

Fig. 8 shows the different curves resulting from measurements of the plethysmography sensors (1.4) in different regions of the user's head. The sensors acquire the pulses from the peripheral extra-cranial blood circulation (6), and identify the presence of blood vessels in the patient's head in the regions wherein the biometric units (1) are positioned. In Fig. 8, three curves are exemplified, and in the first, there is no peripheral circulation in the region wherein the sensor is positioned; in the second, the sensor is positioned exactly on top of the blood vessel, detecting a high level of peripheral circulation; and in the third, the sensor is in a region close to a blood vessel with peripheral circulation, detecting its presence, but at a lower intensity.

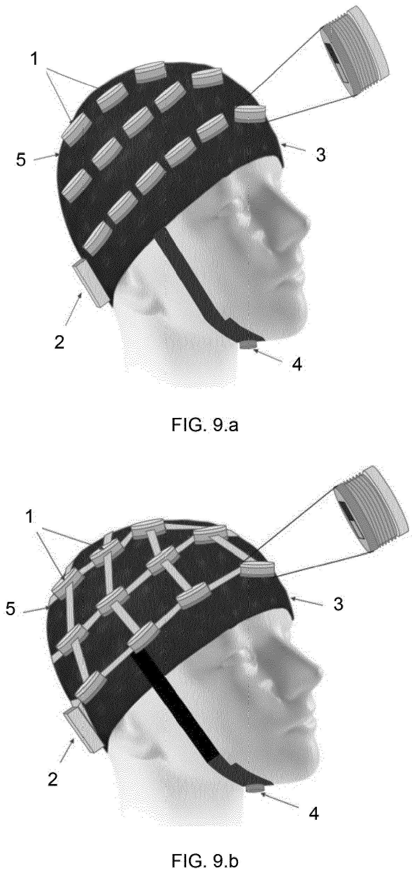

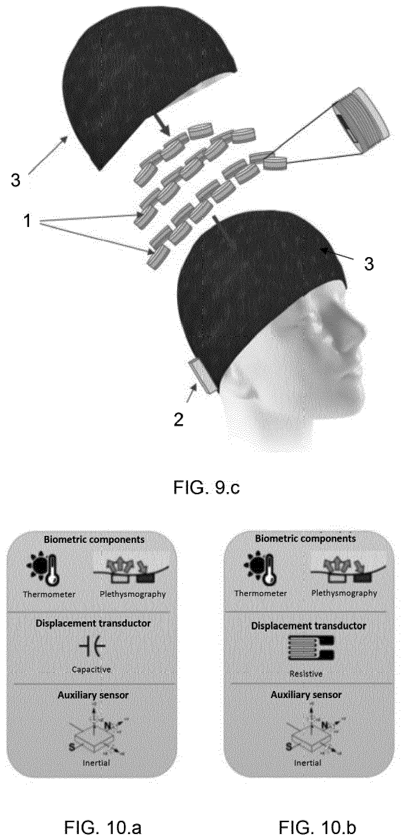

Figs. 9.a to 9.c represent an example wherein a cap is used as a fixation structure (3) in order to obtain a profile of ICP morphology curves along the entire head of the patient, covering a larger area in comparison with a strap, which may appear in other examples. In these figures, it can be seen that the adjustment device (4) is present to safely adjust the cap to the head and prevent movement of the sensors in relation to the body, and the processing unit (2) is still attached to the cap. In Fig. 9.a, the connection between the biometric units (1) and the processing unit (2) is housed under a cap fabric. In Fig. 9.b, the connection between the biometric units (1) and the processing unit (2) is arranged superficially to the cap. In both embodiments, the figures represent an illustrative arrangement for purposes of example and ease of view, since the biometric units (1) are arranged under the cap. In this sense, Fig. 9.c shows an alternative having an additional structure that covers the cap in order to press the biometric units (1) against the patient's head. With that, from the adjustment device (4), it is possible to better position/press the biometric units (1) with said additional structure.

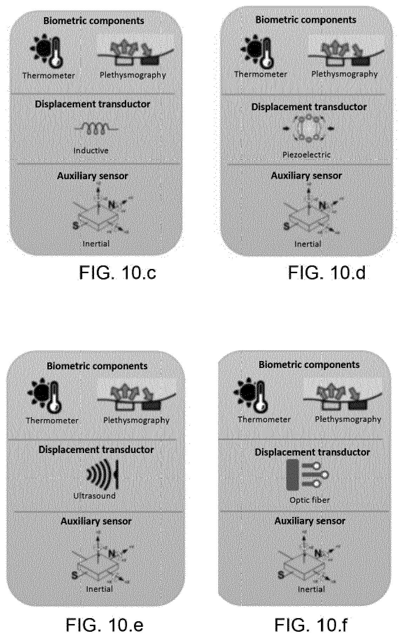

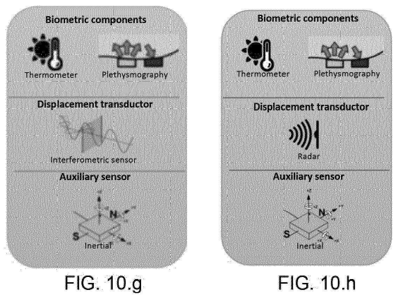

Figs. 10.a to 10.h show an example of all the sensors comprised by each of the biometric units (1). In them, we can observe that, in this case, there is the common acquisition of temperature, as a contact sensor, and plethysmography, as well as the positional characteristics through the IMU. Also, these units are distinguished by the displacement transducer, which can be of capacitive origin in Fig. 10.a, resistive in Fig. 10.b, inductive in Fig. 10.c, piezoelectric in Fig. 10.d, through ultrasound in Fig. 10.e, optical fiber in Fig. 10.f, interferometric in Fig. 10.g and through radar sensor in Fig. 10.h.

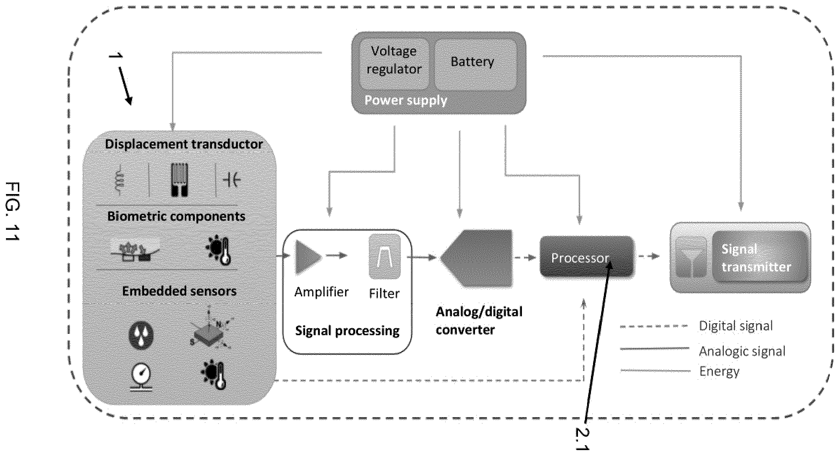

Fig. 11 shows a topology of the device in which there is a source that supplies energy to the system, which is composed of several biometric units (1), which send their signals to a process of amplification and analog filtering of the signal. Then, these signals are converted into digital signals that can be interpreted by the processor (2.1), which in turn sends the processed data to qualified receivers. In this topology, the originally digital signals resulting from the inertial (1.5) and plethysmography (1.4) sensors are sent directly to the processor, with no need to go through the conversion and pre-processing process, due to the technologies used. There is also the alternative that even the signals from the inertial sensors (1.5) and plethysmography (1.4) pass through an analog/digital converter before being directed to the processor (2.1).

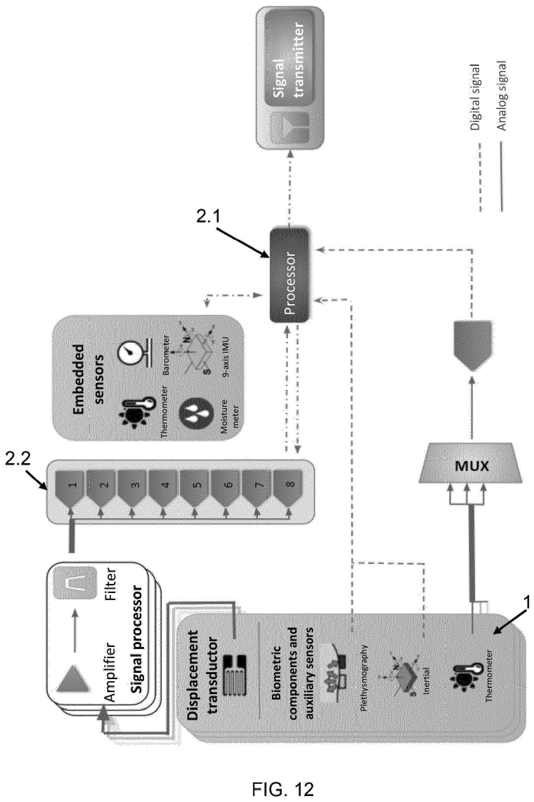

Fig. 12 shows an architecture of the system wherein the topology shown in Fig. 11 is applied, emphasizing the presence of several channels positioned in different locations of the head. Each of the channel signals goes through an analogic processing step, in this case, amplification and filtering, before being sent to the communication interface (2.2). The communication interface (2.2) is provided with multiple analog/digital converters, so that the signals coming from all the biometric units (1) are converted to digital. In this topology of Fig. 12, only the displacement sensor signals (1.1) pass through the analog/digital converters of the communication interface (2.2).

In addition to the signals from the displacement transducers, the processor (2.1) receives digital signals from the plethysmograph, the inertial sensor (1.5), the contact sensor (1.2) from each of the biometric units (1), and additionally receives information from embedded sensors, such as thermometers, barometers, hygrometers and inertial sensors, to capture signals that characterize environmental conditions. All information is acquired and processed in the processor, and sent to the transmitter to send the data to a specific receiver.

Still in this example in Fig. 12, the biometric units are ordered according to the channels, by the processor, so that the displacement (1.1), contact (1.2), plethysmography (1.4) and inertial (1.5) sensors of a given unit biometrics are assigned to a certain channel (e.g. channel 1). Thus, the processor performs a clustering associating all the signals from the different sensors of the respective biometric unit to a given channel, although, as shown in Fig. 12, the contact (1.2), plethysmography (1.4) and inertial (1.5) sensors do not pass through the interface (2.2) together with the displacement sensors (1.1). It should be considered that this example shows one of the countless possibilities of system topology to reproduce the concept of the invention, so as not to limit the objects claimed herein.

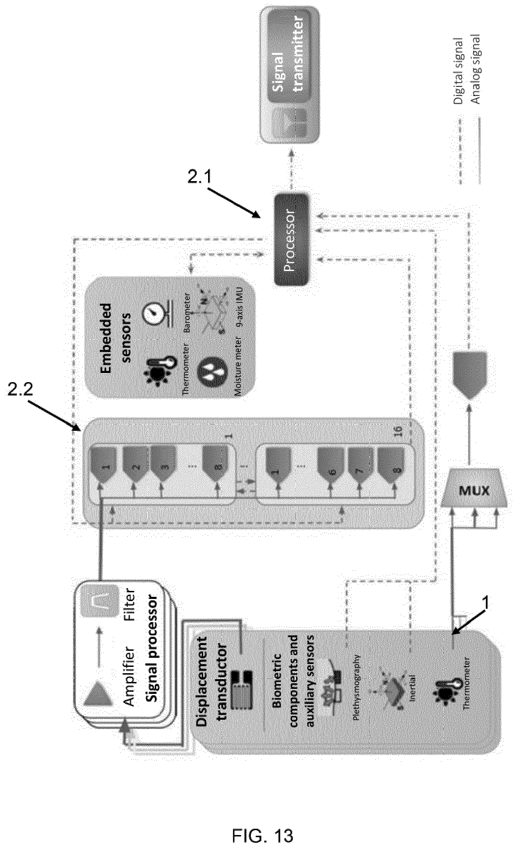

Fig. 13 shows an example similar to Fig. 12, but with the communication interface (2.2) comprising a greater number of analog-to-digital converters, thus forming a Daisy-chain configuration for reading a greater number of analogic channels, obtaining a greater amount of information, with an increase in the number of channels, to generate an optimized ICP morphology curve. In this configuration, 16 8-channel analog/digital converters are capable of synchronously reading up to 128 sensors, including multiple displacement sensors (1.1), auxiliary biometric sensors such as contact sensors (1.2), plethysmography sensors (1.4), inertial sensors (1.5) and ambient sensors.

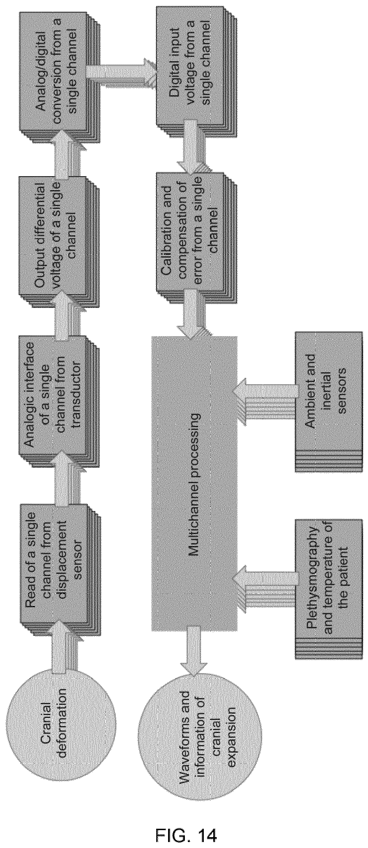

Fig. 14 shows a block diagram that emphasizes the presence of several channels, each representing a plurality of sensors to be acquired and conditioned individually, being sent to the processor to perform the multichannel processing of each of the sensors of each of the channels, with the aim of obtaining a greater amount of information about biometric characteristics and environments that result in obtaining an optimized ICP morphology curve.

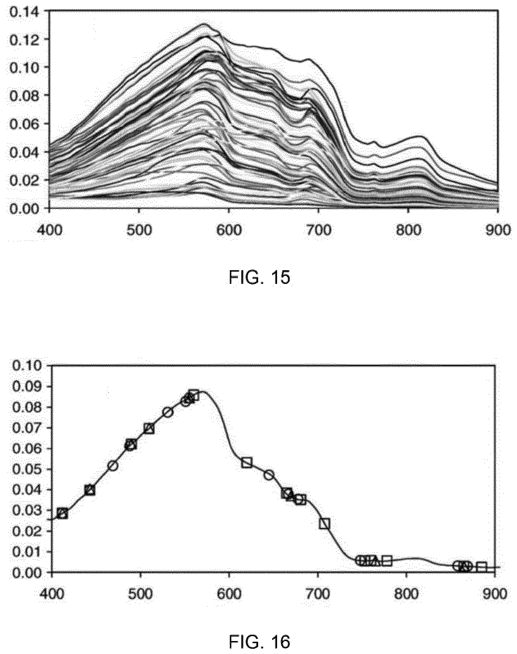

Fig. 15 represents a graph containing all the signals detected by the plurality of biometric units (1) of a patient. In this graph, the displacement signals related to the cranial deformation were processed and represented as displacement versus time.

Fig. 16 represents a graph of an example of statistical processing with the resulting average of all signals that were processed by the processing unit (2), being represented as displacement versus time.

The system of the invention is capable of generating a channel signal quality index, for each of the channels, based on the signals detected by all the biometric units. Said index can be generated by the processor (2.1) or by a component external to the device and may even be under the instruction of a system operator.

Thus, an index is assigned to each channel representing the contribution that the contact, plethysmography and/or movement signals make to the general volumetric variation of the user's skull, in addition to considering the displacement signal itself. The index of each channel is then multiplied by the displacement signals, detected by the displacement sensor (1.1), of the respective channel, so that it is possible to verify the quality of the measurement of the respective channel. The equation below shows an example of the use of these indices:

$$n|CP1 = D1 * QS1 + D2 * QS2 + \ldots + Dn * QSn$$

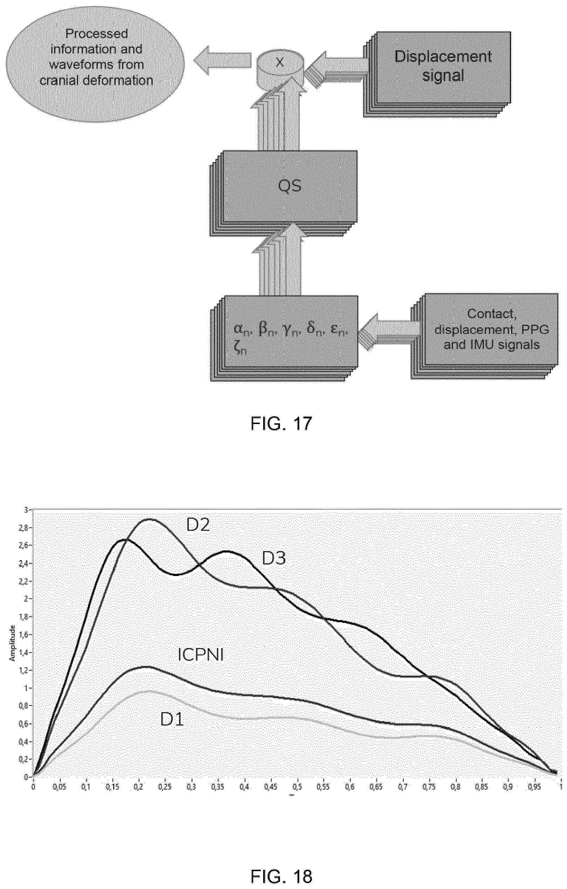

where, $n$ is the channel indicator, $Dn$ is the measurement from the displacement sensor (1.1), and $QSn$ refers to the quality index of channel n, being a non-linear equation represented by the contribution of the contact (1.2), plethysmography (1.4) and inertial (1.5) sensors. The generation of the index can be represented using the flowchart in Fig. 17.

The calculation of the $QSn$ channel quality index takes into account the signals and/or parameters read by the biometric unit n. For representation purposes, in general, the $QSn$ index can be calculated according to the expression below:

$$QSn = a \cdot \alpha_n + b \cdot \beta_n + c \cdot \gamma_n + d \cdot \delta_n + e \cdot \epsilon_n + f \cdot \zeta_n + \cdots$$

where, the components "$\alpha_n$", "$\beta_n$", "$\gamma_n$", "$\delta_n$", "$\epsilon_n$", "$\zeta_n$". etc., are the signals and/or parameters read by the sensors of the biometric unit $n$. The terms "a", "b", "c", "d", "e", "f", etc., are coefficients that can be established theoretically, experimentally and/or with machine learning techniques. In the case of experimental or machine learning, the coefficients are established in preliminary in vivo experiments. It is worth mentioning that this example allows the inclusion of other signs and/or parameters that can be read by the biometric units, not only being restricted to the signs used here.

In an example with displacement, contact, plethysmography and movement signals, the equation for obtaining the $QSn$ quality index can be described below:

$$QSn = \begin{bmatrix} a1 & a2 \end{bmatrix} \begin{bmatrix} PPG_{ampn} \\ PPG_{dcn} \end{bmatrix} + \begin{bmatrix} b1 & b2 \end{bmatrix} \begin{bmatrix} D_{ampn} \\ D_{dcn} \end{bmatrix} + \begin{bmatrix} c1 & c2 & c3 \end{bmatrix} \begin{bmatrix} Gyro_{X_n} \\ Gyro_{Y_n} \\ Gyro_{Z_n} \end{bmatrix}$$

$$+ \begin{bmatrix} d1 & d2 & d3 \end{bmatrix} \begin{bmatrix} Mag_{X_n} \\ Mag_{Y_n} \\ Mag_{Z_n} \end{bmatrix} + \begin{bmatrix} e1 & e2 & e3 \end{bmatrix} \begin{bmatrix} Acc_{X_n} \\ Acc_{Y_n} \\ Acc_{Z_n} \end{bmatrix} + f \cdot Cont_n$$

where, $PPG_{ampn}$: Plethysmography signal amplitude; $PPG_{dcn}$: mean plethysmography signal level; $D_{ampn}$: Amplitude of the displacement signal; $D_{dcn}$: average level of the displacement signal; $Gyro_{Xn}$: gyro X axis signal; $Gyro_{Yn}$: gyroscope Y axis signal; $Gyro_{Zn}$: gyro Z axis signal; $Mag_{Xn}$: magnetometer X axis signal; $Mag_{Yn}$: magnetometer Y axis signal; $Mag_{Zn}$: magnetometer Z axis signal; $Acc_{Xn}$: accelerometer X axis signal; $Acc_{Yn}$: accelerometer Y axis signal; $Acc_{Zn}$: accelerometer Z axis signal; $Cont_n$: contact sensor signal.

There is also an alternative of using non-linear equations to calculate the $QSn$ index, with the coefficients described in the form:

$$a1 = \begin{bmatrix} a1,0 & a1,1 & 1,2 & \cdots & a1,n \end{bmatrix}$$

In the same case, the signals and/or parameters read by the biometric unit can also be written as follows, with only the plethysmography sensor amplitude signal being exemplified, being applicable to all others.

$$PPG_{ampn} = \begin{bmatrix} 1 \\ PPG^1_{ampn} \\ PPG^2_{ampn} \\ \vdots \\ PPG^n_{ampn} \end{bmatrix}$$

Thus, in view of this index, the processor (2.1) or a system operator can select the channels, through the selector element, that have a higher detection quality, that is, that have a lower contribution from contact, plethysmography and/or movement signals, in addition to having less interference from the displacement signal, which can be caused, for example, by a bad positioning of the biometric unit (1) on the user's head. From this, it is possible to obtain the measurement of the cranial deformation in the points that have less interference from these signals.

Thus, the processor (2.1) has a signal compensation tool, which aims to attenuate contributions detected by the contact (1.2), plethysmography (1.4) and inertial (1.5) sensors. Turning to the example in Fig. 8, which shows curves resulting from measurements of plethysmography sensors (1.4) in different regions of the user's head, it is possible to use these measurements to remove components or noise in the measurement.

Through the signal obtained by the plethysmography sensors (1.4) it is possible to infer whether in the ICP morphology signals obtained by the biometric unit (1) there is an additional component of peripheral extra-cranial blood circulation (6). This additional component can have negative effects on non-invasive ICP curves. In an example, wherein a significant contribution of peripheral extra-cranial blood circulation (6) is detected, the influence of the ICP morphology signal obtained from the biometric unit (1) in question is reduced in the final analyzes performed, in order to reduce/subtract the presence of this component from peripheral blood circulation of the ICP morphology.

Said compensation tool, in this example, uses the calculated *QSn* quality indices. Considering only the effect of the plethysmography signal, as shown in Fig. 8, three channels of biometric units (1) were used, where 1 - there is no peripheral circulation; 2 - there is great influence of peripheral circulation; and 3 - moderate influence of the peripheral circulation. In this sense, the *QSn* index can be arbitrarily assigned as follows: *QS*1 = 1.0; *QS*2 = 0; and *QS*3 = 0.1. An exemplary view of the influence of these peripheral circulation signals on the displacement signal can be seen in Fig. 18, where the ICPNI signal represents the morphology of intracranial pressure. Clearly, the signals from D2 and D3 differ from those collected by D1.

In this example it was only addressed the plethysmography signal, however, clearly, this process is applicable to the other signals collected by the biometric units.

To enable the calculations and ratios between the multiple signals of the biometric units (1) of the system, in this case, a processing and synchronization step of the received signals is performed. Different signals can be received at different times and acquisition rates. The communication delay between each sensor and processor (2.1) is compensated so that the signals are temporally synchronized. The signals with the lowest acquisition rate undergo a resampling process through interpolation, increasing the number of points and synchronizing point by point with the signal with the highest acquisition rate.

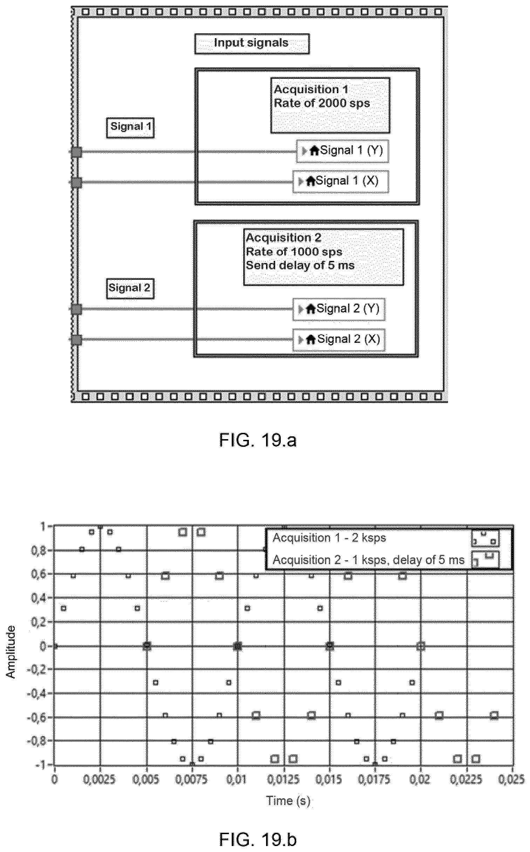

To exemplify and illustrate this step, two signals collected by two sensors with different acquisition rates and with a time delay between them are considered. Signal acquisition is shown through the diagram in Fig. 19.a, wherein the X and Y components of signal 1 being sampled at a rate of 2000 samples/s can be verified, and the X and Y components of signal 2 being sampled at a rate of 1000 samples/s, with a time delay of 5ms. The waveforms can be seen in Fig. 19.b.

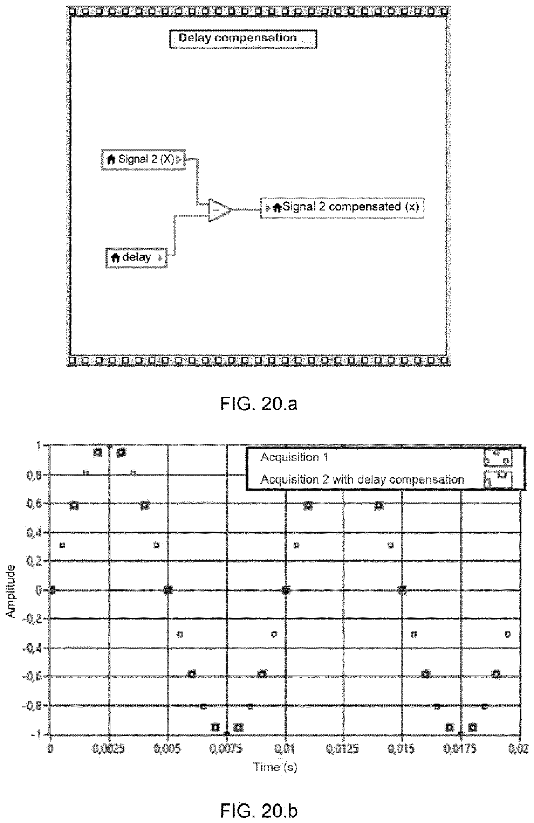

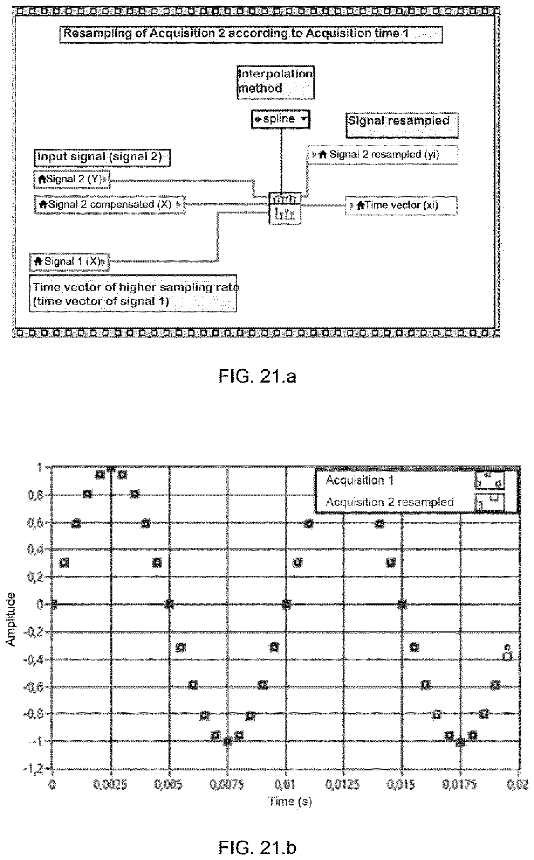

Then, the delay compensation between the two signals is carried out, as can be seen in the diagram in Fig. 20.a and in the waveforms in Fig. 20.b. Thus, after temporal compensation, the signal with the lowest sampling rate undergoes an interpolation, in which case spline is used, so that the other points are calculated, resulting in a signal with the same number of points as the highest sampling rate signal. This step can be verified through the block diagram of Fig. 21.a, with the waveforms illustrated in Fig. 21.b.

In this context, it is immediately clarified that from the disclosure of the present inventive concept, those skilled in the art will be able to consider other ways of realizing the invention not identical to those merely exemplified above, but that in the event of pretense of commercial use such forms may be considered as falling within the scope of the appended claims.

**Claims**

1.  A system for the non-invasive multichannel intracranial pressure detection and monitoring of a user from a cranial deformation, **characterized in that** it comprises:

    a. an intracranial pressure detection and monitoring device comprising a plurality of biometric units (1), positionable on the user's head, which communicates with at least one processing unit (2), said processing unit (2) comprising a processor (2.1) communicating with a communication interface (2.2) for reading and processing signals from each biometric unit (1); and
    b. a receiver operationally communicating with the processing unit (2) of the intracranial pressure detection and monitoring device.

2.  The system of claim 1, **characterized in that** the communication interface (2.2) receives signals from each biometric unit (1), wherein the processor (2.1), by means of a selector element, selects the signal from one or more biometric units (1).

3.  The system of claim 1, **characterized in that** the biometric unit (1) comprises at least one displacement sensor (1.1), at least one contact sensor (1.2), at least one plethysmography sensor (1.4) and at least one an inertial sensor (1.5).

4.  The system of claim 3, **characterized in that** the processor (2.1) comprises a signal compensation tool, which attenuates, from an intracranial pressure signal, signals from the contact sensor (1.2), the plethysmography sensor (1.4) and/or the inertial sensor (1.5).

5.  The system of claim 3, **characterized in that** the processor (2.1) or the receiver comprises a channel signal quality index generation tool, wherein said tool generates a quality index from signals coming from the displacement sensor (1.1), contact sensor (1.2), plethysmography sensor (1.4) and/or inertial sensor (1.5) for each biometric unit (1) of the intracranial pressure detection and monitoring device.

6.  A non-invasive multichannel intracranial pressure detecting and monitoring device, **characterized in that** it comprises:

    a. a plurality of biometric units (1) being in communication with at least one processing unit (2), said processing unit (2) comprising a processor (2.1) communicating with a communication interface (2.2) for reading and processing signals from each biometric unit (1); and
    b. at least one fixation structure (3), positionable on a user's head, wherein the plurality of biometric units (1) are distributed radially along the fixation structure (3).

7.  The multichannel device of claim 6, **characterized in that** the processing unit (2) is positioned on said fixation structure (3).

8.  The multichannel device of claim 6, **characterized in that** the biometric unit (1) comprises at least one displacement sensor (1.1) attached to at least one support of the unit (1.3) that can be associated with the fixation structure (3).

9.  The multichannel device of claim 8, **characterized in that** the biometric unit (1) further comprises at least one contact sensor (1.2), at least one plethysmography sensor (1.4) and at least one inertial sensor (1.5).

10. The multichannel device of claim 9, **characterized in that** the contact sensor (1.2) and the plethysmography sensor (1.4) are arranged in a first region (A), being closer to the user's head.

11. The multichannel device of claim 10, **characterized in that** the first region (A) is opposite the unit support (1.3), wherein the displacement sensor (1.1) is arranged between the first region (A) and the unit support (1.3).

12. A method for non-invasive multichannel intracranial pressure detection and monitoring of a user, from a cranial deformation, **characterized in that** it comprises the steps:

    a. detecting of multiple signals related to the user's cranial deformation, by means of an intracranial pressure detection and monitoring device comprising a plurality of biometric units (1) positionable on the user's head;

b. receiving the multiple signals relating to the cranial deformation by a communication interface (2.2), one or more signals relating to the cranial deformation being sent to a processor (2.1), wherein the communication interface (2.2) and the processor (2.1) are comprised in a processing unit (2); It is

c. processing, by the processor (2.1), one or more signals related to the cranial deformation generating intracranial pressure signals.

13. The method of claim 12, **characterized in that** it further comprises a channel selection step, by means of a selector element, wherein the processor (2.1) selects one or more signals related to the cranial deformation coming from one or more biometric units (1).

14. The method of claim 12, **characterized in that** the biometric unit (1) comprises at least one displacement sensor (1.1), at least one contact sensor (1.2), at least one plethysmography sensor (1.4) and at least one an inertial sensor (1.5).

15. The method of claim 12, **characterized in that** it further comprises a step of generating a channel signal quality index, being generated for each of the multiple signals relating to the cranial deformation, wherein said channel signal quality index is generated from displacement signals, contact signals, plethysmography signals and/or movement signals coming from the plurality of biometric units (1).

FIG. 1

FIG. 2

FIG. 3

FIG. 4

FIG. 5

FIG. 6

FIG. 7.a                    FIG. 7.b

FIG. 8

FIG. 9.a

FIG. 9.b

FIG. 9.c

FIG. 10.a

FIG. 10.b

FIG. 10.c

FIG. 10.d

FIG. 10.e

FIG. 10.f

FIG. 10.g

FIG. 10.h

FIG. 11

FIG. 12

FIG. 13

FIG. 14

FIG. 15

FIG. 16

FIG. 17

FIG. 18

FIG. 19.a

FIG. 19.b

FIG. 20.a

FIG. 20.b

FIG. 21.a

FIG. 21.b

## INTERNATIONAL SEARCH REPORT

| | International application No. |
|---|---|
| | **PCT/BR2021/050456** |

**A. CLASSIFICATION OF SUBJECT MATTER**
IPC: **A61B5/03 (2006.01), A61B5/00 (2006.01)**
CPC: **A61B5/031, A61B5/68, A61B5/6814**
According to International Patent Classification (IPC) or to both national classification and IPC

**B. FIELDS SEARCHED**

Minimum documentation searched (classification system followed by classification symbols)

**A61B**

Documentation searched other than minimum documentation to the extent that such documents are included in the fields searched

**Base de Patentes INPI-BR**

Electronic data base consulted during the international search (name of data base and, where practicable, search terms used)

**DERWENT, GOOGLE PATENTS**

**C. DOCUMENTS CONSIDERED TO BE RELEVANT**

| Category* | Citation of document, with indication, where appropriate, of the relevant passages | Relevant to claim No. |
|---|---|---|
| Y | US 2013018277 A1 (LIU JUNG-TUNG [TW]) 17 JAN 2013 (17. 01.2013) (abstract; Figs. 1 - 6; par. [0010], [0019] - [0023]) | 1 – 15 |
| Y | US 6875176 B2 ( UNIV WASHINGTON [US]) 05 APR 2005 (05.04.2005) (col. 15, 1. 54 - col. 16, 1. 12; col. 19, 1. 37 - 46) | 1 – 15 |
| Y | US 9814426 B2 ( MEDIBOTICS LLC [US]) 14 NOV 2017 (14.11.2017) (abstract; Fig. 7, col. 33, 1. 66 - col. 34, 1. 7; col. 65, 1. 42 - col. 68, 1. 16) | 1 – 15 |
| Y | US 10709345 B1 ( MICHAELI MENASHE [LT]) 14 JUL 2020 (14.07.2020) (abstract; Figs. 1 - 12; col. 2, 1. 55 - col. 4, 1. 14; col. 15, 1. 54 - col. 16, 1. 35) | 1 – 15 |

☒ Further documents are listed in the continuation of Box C.　　☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 10 DEC 2021 (10.12.2021) | 18 DEC 2021 (18.12.2021) |

| Name and mailing address of the ISA/ | Authorized officer |
|---|---|
| ISA/BR | |
| Facsimile No. | Telephone No. |

Form PCT/ISA/210 (second sheet) (January 2015)

**INTERNATIONAL SEARCH REPORT**

International application No.

**PCT/BR2021/050456**

C (Continuation). DOCUMENTS CONSIDERED TO BE RELEVANT

| Category* | Citation of document, with indication, where appropriate, of the relevant passages | Relevant to claim No. |
|---|---|---|
| Y | US 2019175038 A1 (BRAINCARE DESENVOLVIMENTO E INOVACAO TECNOLOGICA S A [BR]) 13 JUN 2019 (13.06.2019) (abstract; Figs. 1-4, 13-19; par. [0015] - [0029], [0051] - [0099]) (from the familly of the cited document in the description) | 1 – 15 |
| Y | US 9993170 B1 (BRAINCARE DESENVOLVIMENTO E INOVACAO TECNOLOGICA LTDA [BR]) 12 JUN 2018 (12.06.2018) (abstract; Figs. 1-3, 16; col. 3, 1. 4 - col. 4, 1. 4; col. 8, 1. 28 - col. 9, 1. 29) (from the familly of the cited document in the description) | 1 – 15 |
| A | US 6740048 B2 (NASA [US]) 25 MAY 2004 (25.05.2004) (abstract; Figs. 1 - 5; col. 3, 1. 60 - col. 6, 67) | 1 – 15 |

Form PCT/ISA/210 (continuation of second sheet) (January 2015)

INTERNATIONAL SEARCH REPORT
Information on patent family members

| International application No. |
|---|
| **PCT/BR2021/050456** |

| | | | |
|---|---|---|---|
| US 2013018277 A1 | 2013-01-17 | None | |
| US 6875176 B2 | 2005-04-05 | None | |
| US 9814426 B2 | 2017-11-14 | None | |
| US 10709345 B1 | 2020-07-14 | US 2021153760 A1 | 2021-05-27 |
| | | US 11058313 B2 | 2021-07-13 |
| | | US 2021338093 A1 | 2021-11-04 |
| | | WO 2021105924 A1 | 2021-06-03 |
| US 2019175038 A1 | 2019-06-13 | AU 2018360391 A1 | 2020-06-18 |
| | | BR 102017023879 A2 | 2019-06-04 |
| | | BR 112020008987 A2 | 2020-10-27 |
| | | CA 3080895 A1 | 2019-05-09 |
| | | CL 2020001157 A1 | 2020-10-23 |
| | | CN 111542263 A | 2020-08-14 |
| | | CO 2020005648 A2 | 2020-05-15 |
| | | EP 3706623 A1 | 2020-09-16 |
| | | IL 274387 D0 | 2020-06-30 |
| | | JP 2021501672 A | 2021-01-21 |
| | | KR 20200086317 A | 2020-07-16 |
| | | PE 20201304 A1 | 2020-11-24 |
| | | WO 2019087148 A1 | 2019-05-09 |
| US 9993170 B1 | 2018-06-12 | BR 112014006364 A2 | 2017-04-04 |
| | | DK 2757939 T3 | 2019-02-18 |
| | | ES 2707884 T3 | 2019-04-05 |
| | | HU E042874 T2 | 2019-07-29 |
| | | PL 2757939 T3 | 2019-05-31 |
| | | US 2013085400 A1 | 2013-04-04 |
| | | US 9826934 B2 | 2017-11-28 |
| | | WO 2013041973 A2 | 2013-03-28 |
| US 6740048 B2 | 2004-05-25 | US 2003191410 A1 | 2003-10-09 |
| | | AU 2002341842 A1 | 2003-10-27 |
| | | AU 2002367864 A1 | 2003-10-27 |
| | | US 2003191411 A1 | 2003-10-09 |
| | | US 6773407 B2 | 2004-08-10 |
| | | WO 03086182 A1 | 2003-10-23 |
| | | WO 03086195 A1 | 2003-10-23 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9202174 A
- US 2009234245 A
- US 2016081608 A
- WO 2013041973 A
- WO 2019087148 A